# EUROPEAN PATENT APPLICATION

(11) **EP 4 710 812 A1**
(43) Date of publication of application: **18.03.2026**
(21) Application number: 24803607.1
(22) Date of filing: 12.04.2024
(51) Int. Cl.: A24F 42/60, A24F 42/20, A24B 15/16, A61M 15/06, A61M 15/00

(54) **INHALER COMPRISING POWDER CARTRIDGE**

(30) Priority: 09.05.2023 KR 20230059703
(71) Applicant: KT&G Corporation, Daedeok-gu Daejeon 34337 (KR)
(72) Inventor: JEOUNG, Eunmi, Daejeon 34128 (KR)
(74) Representative: Ter Meer Steinmeister & Partner
(86) International application number: PCT/KR2024/004890
(87) International publication number: WO 2024/232551

(57) **Abstract**

This inhaler comprises: a casing comprising a first end wall, a second end wall arranged on the opposite side of the first end wall, and a side wall arranged therebetween; a mouthpiece disposed adjacent to the first end wall of the casing; and a cartridge disposed adjacent to the mouthpiece, wherein the cartridge may comprise a rotary screw.

## Description

### TECHNICAL FIELD

The present disclosure relates to an inhaler, and more particularly, to an inhaler including a powder cartridge.

### BACKGROUND ART

Research has been conducted into an inhaler including a reusable cartridge. For example, Korean Patent Application Publication No. 10-2021-0117266 discloses a dry powder inhaler (DPI).

The above description is information the inventor(s) acquired during the course of conceiving the present disclosure, or already possessed at the time, and is not necessarily art publicly known before the present application was filed.

### DISCLOSURE OF THE INVENTION

### TECHNICAL GOALS

One aspect of the present disclosure may deliver a predetermined amount of powder to a user per puff.

One aspect of the present disclosure may increase user usability as a dry powder cartridge may be easily replaced.

### TECHNICAL SOLUTIONS

An inhaler according to various embodiments includes a casing including a first end wall, a second end wall formed opposite to the first end wall, and a side wall formed between the first end wall and the second end wall, a mouthpiece disposed adjacent to the first end wall of the casing, and a cartridge disposed adjacent to the mouthpiece and configured to store functional materials, in which the cartridge is configured to deliver a predetermined amount of the functional materials to the mouthpiece.

The cartridge may further include a housing in which dry powder is stored, a rotation screw disposed inside the housing in a longitudinal direction of the housing, a dispensing cone formed integrally with the rotation screw, in contact with the mouthpiece, and including a plurality of apertures, and a plate disposed between the rotation screw and the dispensing cone and including a powder outlet port, in which disposed inside the housing in the longitudinal direction of the housing, and the dry powder stored in the housing may move to the mouthpiece when the powder outlet port of the plate and the plurality of apertures correspond to each other.

The mouthpiece may include a mouthpiece tip protruding outwardly from the first end wall of the casing, a recessed portion connected to the mouthpiece tip and formed inwardly of the casing, a plurality of partition walls configured to partition a space of the recessed portion, and an elongated cavity extending from the recessed portion to one end of the mouthpiece tip.

The plurality of apertures may correspond to a space formed between the plurality of partition walls that is adjacent to the mouthpiece.

The rotation screw may rotate by 360/n degrees when the number of the plurality of partition walls is n.

The inhaler may further include a puff sensor, in which the rotation screw may rotate about an axis that is parallel to a longitudinal direction of the cartridge when the puff sensor senses a puff of a user.

The inhaler may further include a switch exposed to an outside of the casing, in which the rotation screw may rotate about an axis that is parallel to a longitudinal direction of the cartridge when the switch is clicked.

The mouthpiece may further include a suction delay portion disposed inside of the elongated cavity.

The suction delay portion may be spirally formed in a longitudinal direction of the elongated cavity.

The cartridge may further include a lid disposed on one side of the housing, in which the lid may be opened and closed from the housing.

The cartridge may be reusable.

Additional aspects of embodiments will be set forth in part in the description which follows and, in part, will be apparent from the description, or may be learned by practice of the disclosure.

### EFFECTS OF THE INVENTION

According to an embodiment, an inhaler may deliver a predetermined amount of powder to a user per puff. The inhaler may increase user usability as a dry powder cartridge may be easily replaced. The effects of the inhaler are not limited to the above-mentioned effects, and other unmentioned effects can be clearly understood from the above description by those having ordinary skill in the technical field to which the present disclosure pertains.

### BRIEF DESCRIPTION OF DRAWINGS

The foregoing and other aspects, features, and advantages of embodiments in the disclosure will become apparent from the following detailed description with reference to the accompanying drawings.
FIG. 1 is a perspective view of an inhaler according to an embodiment.
FIG. 2 is an exploded view of the inhaler according to an embodiment.
FIG. 3 is a perspective view of a cartridge according to an embodiment.
FIG. 4 is a cross-sectional view of the inhaler according to an embodiment.
FIG. 5 is a perspective view of a mouthpiece according to an embodiment.
FIG. 6 is a cross-sectional view of the mouthpiece according to an embodiment.

### BEST MODE FOR CARRYING OUT THE INVENTION

The terms used in the embodiments are selected from among common terms that are currently widely used, in consideration of their function in the disclosure. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, the expression "at least one of a, b, or c" should be construed as including a, b, c, a and b, a and c, b and c, or a, b, and c.

In the following embodiments, an "inhaler" may include a suction material in the form of powder or liquid and may refer to an article that delivers the suction material to the oral region of a user. A representative example of the inhaler may be a dry powder inhaler (DPI). However, the scope of the present disclosure is not limited thereto.

In the following embodiments, the term "upstream" or "upstream direction" may refer to a direction away from the oral region of a user (smoker), and the term "downstream" or "downstream direction" may refer to a direction towards the oral region of the user. The terms "upstream" and "downstream" may be used to describe relative positions of components of an inhaler.

In the following embodiments, the term "puff" refers to inhalation by a user, and the inhalation refers to a situation in which a user draws in an aerosol into their oral cavity, nasal cavity, or lungs through the mouth or nose.

Hereinafter, embodiments of the disclosure are described in detail with reference to the diagrams.

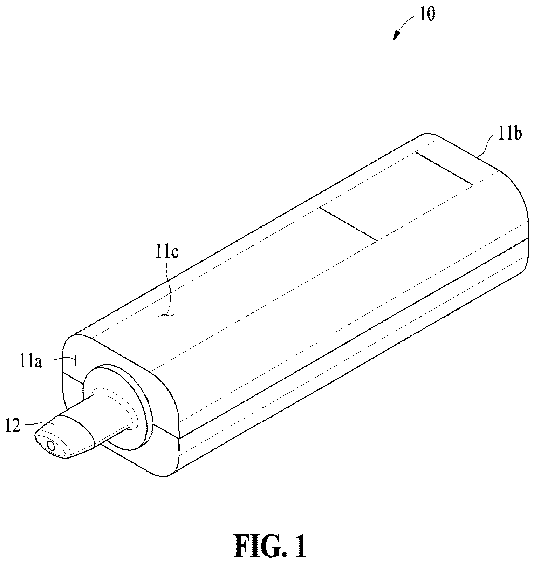

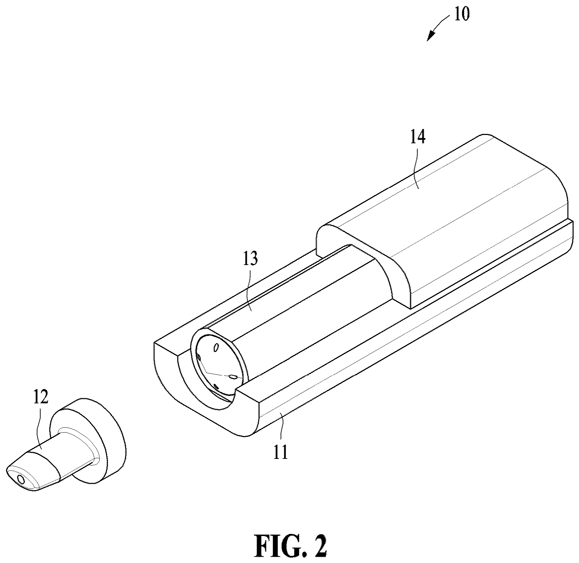

FIG. 1 is a perspective view of an inhaler 10 according to an embodiment. FIG. 2 is an exploded view of the inhaler 10 according to an embodiment.

Referring to FIGS. 1 and 2, the inhaler 10 may include a casing 11, a mouthpiece 12, a cartridge 13, and a driving unit 14.

In an embodiment, the casing 11 may form the exterior of the inhaler 10. The casing 11 may cover components accommodated in the casing 11. The casing 11 may be made of a material with high strength and rigidity, so the casing 11 may protect the components from shock, heat, dust, dirt, etc., delivered from the outside. The casing 11 may include a first end wall 11a, a second end wall 11b, and a side wall 11c. The first end wall 11a may be a wall formed in a direction on which the mouthpiece 12 is disposed. The second end wall 11b may be a wall disposed on the opposite side of the first end wall 11a. The side wall 11c may be a wall formed between the first end wall 11a and the second end wall 11b. Powder stored in the inhaler 10 may move in a direction from the second end wall 11b toward the first end wall 11a.

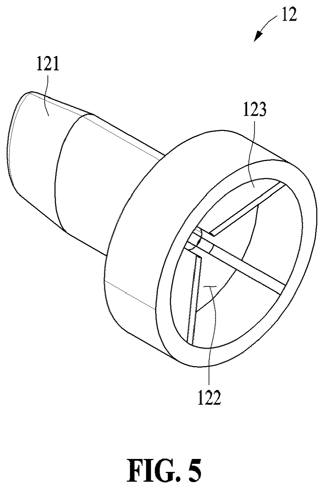

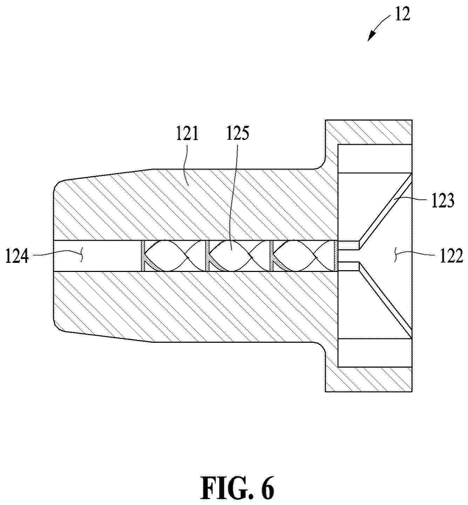

In an embodiment, the mouthpiece 12 may contact the oral region of a user when the user inhales a suction material stored in the inhaler 10. The mouthpiece 12 may be disposed adjacent to the first end wall 11a of the casing 11. The mouthpiece 12 may be formed protruding from the first end wall 11a. The mouthpiece 12 according to an embodiment is described in more detail below with reference to FIGS. 5 and 6.

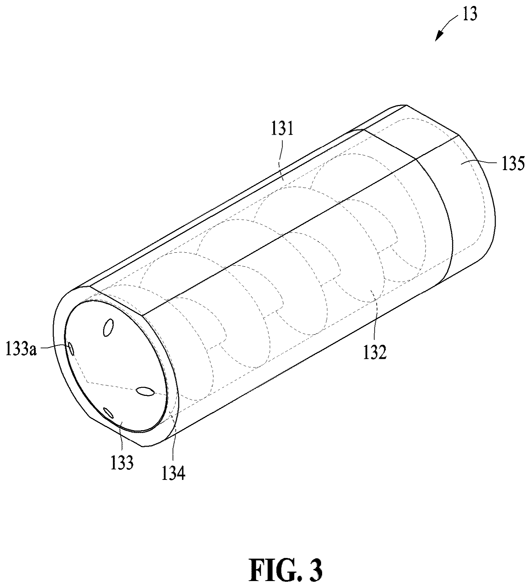

In an embodiment, the cartridge 13 may be accommodated in the casing 11. The cartridge 13 may be disposed adjacent to the mouthpiece 12. The cartridge 13 may accommodate a functional material that a user desires to inhale. For example, the functional material may include at least one of nicotine, theanine, caffeine, taurine, pharmaceutical materials, or a mixture thereof. The functional material may be in the form of fine granules or dry powder. As the functional material stored in the cartridge 13 is delivered to the mouthpiece 12, the user may inhale the functional material. The cartridge 13 according to an embodiment is described in more detail below with reference to FIG. 3.

In an embodiment, the driving unit 14 may be disposed adjacent to the cartridge 13. For example, the driving unit 14 may be disposed downstream of the cartridge 13. The driving unit 14 may include a battery (not shown) and a controller (not shown).

The battery may supply power used to operate the inhaler 10. For example, a rotation screw (e.g., a rotation screw 132 of FIG. 3) of the cartridge 13 may rotate by receiving the power from the battery. The controller may control the overall operation of the inhaler 10. In an embodiment, the controller may include at least one processor. The at least one processor may be implemented as an array of a plurality of logic gates or may be implemented as a combination of a general-purpose microprocessor and a memory in which a program executable by the microprocessor is stored. In addition, it may be understood by those having ordinary skill in the art to which the present disclosure pertains that it may be implemented in other types of hardware.

Although not shown in FIG. 1 and/or FIG. 2, the inhaler 10 may further include a puff sensor or a switch. In an embodiment, the inhaler 10 including the puff sensor may recognize inhalation of a user and may cause the rotation screw 132 to rotate without a separate input. The puff sensor may sense the inhalation (puff) of the user. When the puff sensor senses the inhalation of the user, a signal may be transmitted to the controller of the driving unit 14, so the rotation screw (e.g., the rotation screw 132 of FIG. 3) may rotate. The rotation screw 132 may rotate about an axis that is parallel to the longitudinal direction of the cartridge 13. In an embodiment, the inhaler 10 including the switch may cause the rotation screw 132 to rotate when the user clicks (inputs) the switch. When the user clicks the switch, a signal may be transmitted to the controller of the driving unit 14, so the rotation screw 132 may rotate.

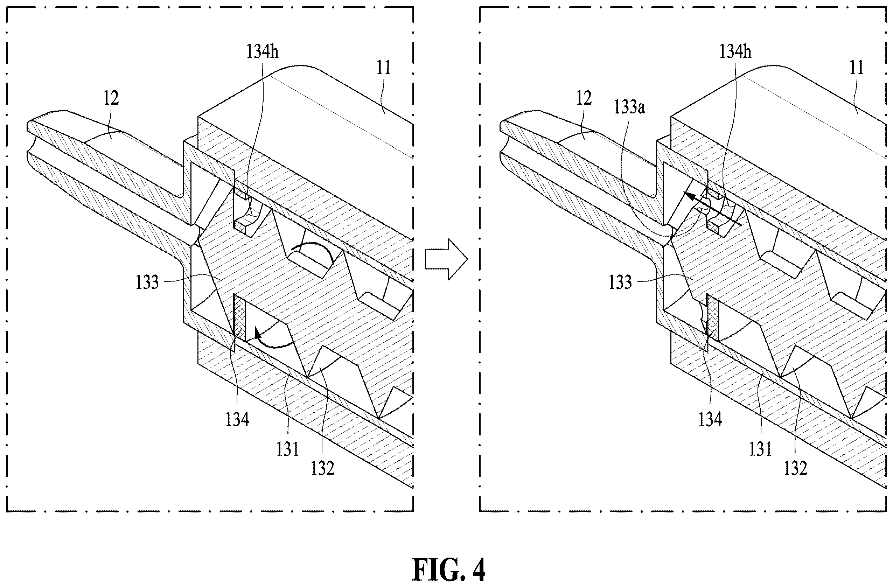

FIG. 3 is a perspective view of the cartridge 13 according to an embodiment. FIG. 4 is a cross-sectional view of the inhaler 10 according to an embodiment.

FIGS. 3 and 4 show an internal structure and driving mechanism of the cartridge 13. The cartridge 13 may include a housing 131, the rotation screw 132, a dispensing cone 133, a plate 134, and a lid 135.

In an embodiment, the housing 131 may form the exterior of the cartridge 13. The housing 131 may have a shape corresponding to the internal space of the casing 11 of an inhaler (e.g., the inhaler 10 of FIG. 1). For example, the housing 131 may have a cylindrical shape. The housing 131 may store functional materials (e.g., dry powder and the like) accommodated in the housing 131. The housing 131 may be made of a material with high strength and rigidity, so the housing 131 may protect components from shock, heat, dust, dirt, etc., delivered from the outside.

In an embodiment, the rotation screw 132 may include a thread formed about an axis formed in the longitudinal direction of the cartridge 13. As the rotation screw 132 rotates, the thread of the rotation screw 132 may move forward. A corner of the thread of the rotation screw 132 may contact the inner surface of the housing 131. As the corner of the thread contacts the inner surface of the housing 131, the delivery efficiency may be maximized when the functional materials are pushed toward the dispensing cone 133 as the rotation screw 132 rotates. The rotation screw 132 may be formed from one end portion to the other end portion in the longitudinal direction of the cartridge 13.

In an embodiment, the dispensing cone 133 may be disposed on one end portion of the cartridge 13. The dispensing cone 133 may contact the mouthpiece 12. For example, the dispensing cone 133 may contact a partition wall (e.g., a partition wall 123 of FIGS. 5 and 6) of the mouthpiece 12. The dispensing cone 133 may be formed integrally with the rotation screw 132 (see FIG. 4). As the dispensing cone 133 is formed integrally with the rotation screw 132, the dispensing cone 133 may rotate together with the rotation screw 132. The dispensing cone 133 may include an aperture 133a. The aperture 133a of the dispensing cone 133 may be formed in plurality. The aperture 133a may be formed to communicate with the internal space of the cartridge 13 and the external space. The aperture 133a may be formed at a predetermined angular interval at a position that is adjacent to an edge of the dispensing cone 133. For example, when the dispensing cone 133 includes four apertures 133a, each aperture 133a may be disposed at a 90 degrees (°) interval. For example, when the dispensing cone 133 includes three apertures 133a, each aperture 133a may be disposed at a 120° interval.

In an embodiment, the plate 134 may be disposed between the rotation screw 132 and the dispensing cone 133. That is, the plate 134 may include a large hole formed in the center of the plate 134 and the rotation screw 132 and the dispensing cone 133 may be formed integrally through the large hole. The plate 134 may be formed separately from the rotation screw 132 and the dispensing cone 133. The plate 134 may be fixed to the housing 131 even when the rotation screw 132 rotates. The plate 134 may include a powder outlet port 134h. The powder outlet port 134h may be formed to penetrate the cartridge 13 in the longitudinal direction. The diameter of the powder outlet port 134h may be greater than that of the aperture 133a of the dispensing cone 133. As the rotation screw 132 rotates, when one of a plurality of apertures 133a of the dispensing cone 133 corresponds to the powder outlet port 134h, the dry powder stored in the cartridge 13 may move to the mouthpiece 12 (see the arrow of FIG. 4).

In an embodiment, the lid 135 may be formed on one side of the housing 131 of the cartridge 13. The lid 135 may be separated (opened and closed) from the housing 131. As the cartridge 13 is configured to be opened and closed through the lid 135, the functional materials may be recharged by opening the lid 135 when the functional materials stored in the cartridge 13 are depleted. Accordingly, the cartridge 13 according to an embodiment may be reusable.

FIG. 5 is a perspective view of the mouthpiece 12 according to an embodiment. FIG. 6 is a cross-sectional view of the mouthpiece 12 according to an embodiment.

Referring to FIGS. 5 and 6, the mouthpiece 12 may include a mouthpiece tip 121, a recessed portion 122, a partition wall 123, an elongated cavity 124, and a suction delay portion 125.

In an embodiment, the mouthpiece tip 121 may correspond to a part directly contacting the oral region of a user. The mouthpiece tip 121 may be formed to protrude outwardly from a first end wall (e.g., the first end wall 11a of FIG. 1) of a casing (e.g., the casing 11 of FIG. 1). The mouthpiece tip 121 may be formed such that the thickness decreases as the mouthpiece tip 121 moves from upstream to downstream. The mouthpiece tip 121 may be formed flat in a direction that is perpendicular to the longitudinal direction. The mouthpiece tip 121 may include the elongated cavity 124 formed in the longitudinal direction inside the mouthpiece tip 121.

In an embodiment, the recessed portion 122 may be formed on the opposite side of the mouthpiece tip 121. The recessed portion 122 may be formed integrally with the mouthpiece tip 121. The recessed portion 122 may include a space formed inwardly of a casing (e.g., the casing 11 of FIG. 4). The recessed portion 122 may temporarily store a functional material (e.g., dry powder) that passes through the powder outlet port 134h of the plate 134 and the aperture 133a of the dispensing cone 133 (see FIG. 4). The recessed portion 122 may communicate with the elongated cavity 124 formed in the longitudinal direction inside the mouthpiece tip 121.

In an embodiment, the partition wall 123 may be formed to protrude from an edge of the recessed portion 122 toward the center of the recessed portion 122. The partition wall 123 may be formed in plurality. A plurality of partition walls 123 may not contact each other and may extend to the start point of the elongated cavity 124. The plurality of partition walls 123 may partition the internal space of the recessed portion 122. The space formed between the partition walls 123 that are adjacent to each other may correspond to the aperture 133a formed in the dispensing cone 133 of the cartridge 13. The functional material that moves to the mouthpiece 12 through the aperture 133a may be temporarily stored in the space formed between the partition walls 123 of the mouthpiece 12 and then may move to the oral region of a user through the elongated cavity 124. The partition walls 123 may be formed at a predetermined angular interval in the recessed portion 122. For example, when the recessed portion 122 includes four partition walls 123, each partition wall 123 may be disposed at a 90° interval. For example, when the recessed portion 122 includes three partition walls 123, each partition wall 123 may be disposed at a 120° interval. That is, when the number of partition walls 123 formed in the recessed portion 122 is n, it may be desirable that the number of apertures (e.g., the aperture 133a of FIG. 3) formed in a dispensing cone (e.g., the dispensing cone 133 of FIG. 3) of the cartridge 13 is n, and a rotation screw (e.g., the rotation screw 132 of FIG. 3) may rotate by 360/n degrees per puff or switch click of the user.

In an embodiment, the elongated cavity 124 may extend from the center of the recessed portion 122 to one end of the mouthpiece tip 121 and may fluidly communicate with the outside of the inhaler 10. The functional material temporarily stored in the recessed portion 122 may move to the oral region of a user through the elongated cavity 124.

In an embodiment, a suction delay portion 125 may be formed inside the elongated cavity 124. The suction delay portion 125 may prevent a large amount of functional materials from moving to the oral region of a user at once due to one puff by the user. The suction delay portion 125 may form a structure having a complex shape inside the elongated cavity 124 formed straight in the longitudinal direction. For example, the suction delay portion 125 may include a spiral structure that rotates around an axis that is parallel to the longitudinal direction of the elongated cavity 124. Due to the complex shape of the suction delay portion 125, it may be possible to prevent a large amount of functional materials (e.g., dry powder) from being inhaled at once due to a long flow path and causing a user's cough or the like.

While the examples are described with reference to drawings, it will be apparent to one of ordinary skill in the art that various alterations and modifications in form and details may be made in these examples without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and/or equivalents of the claims are within the scope of the following claims.

## Claims

1. An inhaler comprising:
a casing comprising a first end wall, a second end wall formed opposite to the first end wall, and a side wall formed between the first end wall and the second end wall;
a mouthpiece disposed adjacent to the first end wall of the casing; and
a cartridge disposed adjacent to the mouthpiece and configured to store functional materials,
wherein the cartridge is configured to deliver a predetermined amount of the functional materials to the mouthpiece.

2. The inhaler of claim 1, wherein the cartridge further comprises:
a housing in which dry powder is stored;
a rotation screw disposed inside the housing in a longitudinal direction of the cartridge;
a dispensing cone formed integrally with the rotation screw, in contact with the mouthpiece, and comprising a plurality of apertures; and
a plate disposed between the rotation screw and the dispensing cone and comprising a powder outlet port,
wherein the rotation screw is disposed inside the housing in the longitudinal direction of the cartridge, and
wherein the dry powder stored in the housing moves to the mouthpiece when the powder outlet port of the plate and the plurality of apertures correspond to each other.

3. The inhaler of claim 2, wherein the mouthpiece comprises:
a mouthpiece tip protruding outwardly from the first end wall of the casing;
a recessed portion connected to the mouthpiece tip and formed inwardly of the casing;
a plurality of partition walls configured to partition a space of the recessed portion; and
an elongated cavity extending from the recessed portion to one end of the mouthpiece tip.

4. The inhaler of claim 3, wherein the plurality of apertures corresponds to a space formed between the plurality of partition walls that is adjacent to the mouthpiece.

5. The inhaler of claim 4, wherein the rotation screw rotates by 360/n degrees when a number of the plurality of partition walls is n.

6. The inhaler of claim 2, further comprising:
a puff sensor, and
wherein the rotation screw rotates about an axis that is parallel to a longitudinal direction of the cartridge when the puff sensor senses a puff of a user.

7. The inhaler of claim 2, further comprising:
a switch exposed to an outside of the casing, and
wherein the rotation screw rotates about an axis that is parallel to a longitudinal direction of the cartridge when the switch is clicked.

8. The inhaler of claim 3, wherein the mouthpiece further comprises a suction delay portion disposed inside of the elongated cavity.

9. The inhaler of claim 8, wherein the suction delay portion is spirally formed in a longitudinal direction of the elongated cavity.

10. The inhaler of claim 2, wherein the cartridge further comprises a lid disposed on one side of the housing, and
wherein the lid is capable of being opened and closed from the housing.

11. The inhaler of claim 1, wherein the cartridge is reusable.
